# EUROPÄISCHE PATENTSCHRIFT

(11) **EP 4 119 231 B1**
(45) Veröffentlichungstag und Bekanntmachung des Hinweises auf die Patenterteilung: **15.01.2025**
(21) Anmeldenummer: 21185230.6
(22) Anmeldetag: 13.07.2021
(51) Int. Cl.: B05B 11/10, A61M 15/00, A61M 15/08, A61M 11/00, B05B 1/14

(54) **FLÜSSIGKEITSSPENDER**
LIQUID DISPENSER
DISTRIBUTEUR DE LIQUIDE

(43) Veröffentlichungstag der Anmeldung: 18.01.2023
(73) Patentinhaber: Aptar Radolfzell GmbH, 78315 Radolfzell (DE)
(72) Erfinder: Ritsche, Stefan, 78253 Eigeltingen (DE)
(74) Vertreter: Witte, Weller und Partner Patentanwälte mbB Stuttgart

(56) Entgegenhaltungen:
- WO-A1-2004/108301
- WO-A1-2009/077013
- WO-A1-2012/127167
- WO-A1-2015/091936
- WO-A1-2020/074254
- DE-A1- 102010 018 964
- US-A1- 2017 361 344

## Beschreibung

### ANWENDUNGSGEBIET UND STAND DER TECHNIK

Die Erfindung betrifft einen Flüssigkeitsspender zum Austrag von einer pharmazeutischen Flüssigkeit oder von einer Nikotin und/oder Cannabis enthaltenden Flüssigkeit. Die Erfindung betrifft darüber hinaus ein Set, welches einen solchen Flüssigkeitsspender umfasst.

Gattungsgemäße Flüssigkeitsspenderweisen ebenso wie auch erfindungsgemäße Flüssigkeitsspender einen Flüssigkeitsspeicher sowie eine damit koppelbare oder gekoppelte Austragvorrichtung auf, wobei die Austragvorrichtung über eine Basis und eine demgegenüber zum Zweck der Betätigung einer Pumpeinrichtung bewegliche Betätigungshandhabe verfügt. Wird die Pumpeinrichtung betätigt, so wird Flüssigkeit aus dem Flüssigkeitsspeicher zu einer Austragöffnung gefördert und abgegeben. Solche Flüssigkeitsspender sind üblicherweise für einen Applikationsweg, beispielsweise die orale oder nasale Applikation ausgebildet, beispielsweise durch einen Nasalapplikator zur Einführung in ein Nasenloch des Nutzers.

Bekannte Flüssigkeitsspender sind meist als Einwegprodukte ausgebildet. Dies bedeutet, dass vergleichsweise viel Material und insbesondere häufig in Kombination auch nicht ideal gemeinsam recyclebares Material entsorgt werden muss, wenn der Flüssigkeitsspender entleert wurde. Die übliche Gestaltung als Einwegprodukt begrenzt darüber hinaus die Kosten des Flüssigkeitsspenders, so dass technisch oder ästhetisch vorteilhafte Verbesserungen aus wirtschaftlichen Gründen mitunter nicht umsetzbar sind.

Aus der WO 2004/108301 A1, der WO 2015/091936 A1, der WO 2012/127167 A1 sowie der WO 2009/077013 A1 und der US 2017/0361344 A1 sind jeweils Spender bekannt, die über jeweils zwei Teileinheit verfügen, von denen eine die Basis und die demgegenüber bewegliche Betätigungshandhabe bereitstellen und von denen die andere den Flüssigkeitsspeicher und die Pumpeinrichtung umfassen. Eine ähnliche Bauweise findet sich auch in der DE 10 2010 018 964 A1.

### AUFGABE UND LÖSUNG

Aufgabe der Erfindung ist es, einen Flüssigkeitsspender bekannter Art dahingehend weiterzubilden, dass die Teileinheit, die die Pumpeinrichtung und den Flüssigkeitsspeicher umfasst, keine Gefahr für Kinder darstellt.

Erfindungsgemäß wird zur Lösung dieser Aufgabe ein Flüssigkeitsspender vorgeschlagen, der dem Austrag einer pharmazeutischen Flüssigkeit oder einer Nikotin und/oder Cannabis enthaltenden Flüssigkeit dient, die vor dem Austrag in einem Flüssigkeitsspeicher des Flüssigkeitsspenders gelagert ist und die durch mindestens eine Austragöffnung ausgetragen werden kann.

Die mindestens eine Austragöffnung kann je nach Anwendungsfall unterschiedlich ausgebildet sein, beispielsweise mit einer vorgeschalteten Wirbelkammer zur Zerstäubung der Flüssigkeit oder mit einem Düsenelement mit einer Düsenplatte, in dem eine Vielzahl von kleinen Öffnungen zur Erzeugung eines feinen Aerosols vorgesehen sind. Weiterhin kann die mindestens eine Austragöffnung je nach Anwendungszweck am distalen Ende eines Nasalapplikators oder auch an einem Applikator zur oralen Einnahme der Flüssigkeit vorgesehen sein.

Neben dem Flüssigkeitsspeicher, der vorzugsweise ein Volumen zwischen 5 ml und 50 ml, insbesondere zwischen 10 ml und 20 ml, aufweist, umfasst der Flüssigkeitsspender eine Austragvorrichtung, die eine zumindest mittelbar mit dem Flüssigkeitsspeicher gekoppelte Basis sowie eine gegenüber dieser Basis bewegliche Betätigungshandhabe verfügt. Die Basis dient zur Betätigung einer Pumpeinrichtung des Flüssigkeitsspenders. Diese Pumpeinrichtung weist in üblicher Weise eine Pumpkammer auf, die eingangsseitig und ausgangsseitig durch jeweils ein Ventil begrenzt ist, so dass eine Verkleinerung der Pumpkammer das eingangsseitige Ventil schließt und durch das ausgangsseitige Ventil Flüssigkeit zu einer Austragöffnung drückt. Bei einer Vergrößerungder Pumpkammer schließt das ausgangsseitige Ventil und Flüssigkeit aus dem Flüssigkeitsspeicher wird in die Pumpkammer gesogen. Die volumetrische Veränderlichkeit der Pumpkammer kann insbesondere durch einen an Wandungen der Pumpkammer dichtend gleitenden Kolben erzielt werden. Eine andere mögliche Bauform sieht eine bewegliche Membran vor, die gegenüber den anderen Wandungen der Pumpkammerverlagerbarist und so eine Vergrößerung und Verkleinerung der Pumpkammer zum Zwecke des Ansaugens und Herausdrückens von Flüssigkeit ermöglicht.

Erfindungsgemäß ist vorgesehen, dass der Flüssigkeitsspender als modularer Flüssigkeitsspender ausgebildet ist, der zwei Teileinheiten aufweist, nämlich eine erste und eine zweite Teileinheit. Diese beiden Teileinheiten werden bestimmungsgemäß vom Endverbraucher selbst gehandhabt, also voneinander getrennt und zusammengesetzt. Beide Teileinheiten können bestimmungsgemäß mit verschiedenen anderen Teileinheiten verbunden werden, wobei der Zweck dessen im Weiteren noch erläutert wird.

Die erste Teileinheit umfasst den Flüssigkeitsspeicher und die hiermit verbundene Pumpeinrichtung, wobei die Pumpeinrichtungvorzugsweise derart hermetisch abgedichtet am Speicherbehälter des Flüssigkeitsspeichers angebracht ist, dass Flüssigkeit nur durch die Pumpeinrichtung hindurch aus dem Flüssigkeitsspeicher austreten kann. Eine werkzeuglose Trennung der Pumpeinrichtung vom Speicherbehälter des Flüssigkeitsspenders ist üblicherweise nicht gewollt und ist daher vorzugsweise baulich unterbunden, bspw. durch eine einstückige Gestaltung des Pumpengehäuses mit dem Speicherbehälter oder durch eine nicht zerstörungsfrei trennbare Verrastung.

Die zweite Teileinheit weist die Austragvorrichtung und damit die Basis, die demgegenüber bewegliche Betätigungshandhabe sowie die mindestens eine Austragöffnung auf. Die zweite Teileinheit weist weiterhin einen Einlass auf, der der Schaffung einer Fluidverbindung mit dem Auslass der ersten Teileinheit dient. Im gekoppelten Zustand ist dieser Einlass stromabwärts der Pumpeinrichtung der ersten Teileinheit vorgesehen.

Die beiden Teileinheiten verfügen über zusammenwirkende Kopplungsteileinrichtungen einer Kopplungseinrichtung, um werkzeuglos miteinander gekoppelt und voneinander entkoppelt werden zu können. Diese Kopplung bewirktzum einen die genannte Fluidverbindung, durch die der Einlass der zweiten Teileinheit und damit mittelbar auch die mindestens eine Austragöffnung mit der Pumpeinrichtung verbunden sind. Zum anderen ist diese Kopplung eine mechanische Kopplung, durch die die beiden Teileinheiten gemeinsam gehandhabt werden können und insbesondere die Basis der zweiten Teileinheit und der Speicherbehälter der ersten Teileinheit in fester Relativstellungzueinander gesichert sind.

Die beiden Teileinheiten sind dafür ausgebildet, einfach miteinander koppelbarzu sein und einfach voneinander getrennt werden zu können, so dass der Endanwender dies problemlos und im besten Falle mit nur einem Handgriff bewerkstelligen kann. Im gekoppelten Zustand ist der Spender in üblicher Weise verwendbar. Wird die Betätigungshandhabe bedient, bspw. heruntergedrückt, wird die entsprechende Kraft direkt oder über ein Getriebe an die Pumpeinrichtung der ersten Teileinheit weitergeleitet und betätigt diese, so dass Flüssigkeit aus der Pumpkammer in die zweite Teileinheit und somit in Richtung der Austragöffnung gefördert wird.

Durch die einfache Koppelbarkeit und Trennbarkeit der Teileinheiten ist es möglich, die gleiche zweite Teileinheit mit unterschiedlichen ersten Teileinheiten zu verwenden. Wenn der Flüssigkeitsspeicher der ersten Teileinheit entleert wurde, kann dieser somit zusammen mit der Pumpeinrichtung entsorgt werden, während die zweite Teileinheit mit einer neuen ersten Teileinheit und damit einem befüllten Flüssigkeitsspeicher ergänzt wird. Dies gestattet es unter anderem, einen höheren Herstellungsaufwand hinsichtlich der zweiten Teileinheit in Kauf zu nehmen. So kann diese beispielsweise teilweise metallisch oder aus vergleichsweise edlen und ästhetisch oder haptisch vorteilhaften Kunststoffen gebildet sein. Auch wird es insbesondere hierdurch wirtschaftlich sinnvoll, im Bereich der Austragöffnung eine komplexere und damit teurere Gestaltung vorzusehen, beispielsweise eine Düsenplatte mit einer Vielzahl von Düsenlöchern zur Erzeugung eines feinen Aerosols. Bei Einwegspendern ist die Verwendung einer solchen Düsenplatte häufig zu teuer. Ein weiterer Aspekt, der insbesondere bei der mehrfachen Verwendung der zweiten Teileinheit mit wechselnden ersten Teileinheiten wirtschaftlich sinnvoll ist, ist die Ausgestaltung der zweiten Teileinheit mit einer elektronischen Erfassungseinrichtung zur Erfassung der Handhabung des Flüssigkeitsspenders. Eine solche Erfassungseinrichtung umfasst üblicherweise mindestens eine Batterie oder einen Stromgenerator, einen integrierten Schaltkreis oder Prozessor sowie eine Sensorik. Die damit einhergehenden Kosten können insbesondere bei einer zweiten Teileinheit berechtigt sein, die über einen langen Zeitraum verwendet wird. Die Erfassungseinrichtung ist vorzugsweise dafür ausgebildet, eine Betätigung der Betätigungshandhabe und/oder den Austrag von Flüssigkeit zu erfassen.

Ein weiterer Vorteil, der sich bei der wiederholten Verwendung der zweiten Teileinheit ergibt, wenn diese in erfindungsgemäßer Weise ohne Pumpeinrichtung ausgebildet ist, liegt in der einfachen Reinigung. Die zweite Teileinheit weist vorzugsweise lediglich einen sehr einfachen Flüssigkeitspfad vom Einlass bis zur Auslassöffnung auf und kann daher einfach gereinigt und insbesondere gespült werden.

Die Verwendung der gleichen zweiten Teileinheit mit mehreren ersten Teileinheiten ist nicht nur sinnvoll, wenn ein leerer Flüssigkeitsspeicher gegen einen vollen ausgetauscht werden soll. Es kann auch zweckmäßig sein, um mit der gleichen zweiten Teileinheit unterschiedliche Flüssigkeiten auszutragen. Im Falle der Verwendung eines erfindungsgemäßen Spenders als Nikotinspray kann somit beispielsweise zwischen unterschiedlichen Geschmacksrichtungen oder Stärken gewählt werden.

Die erfindungsgemäße Gestaltung des Flüssigkeitsspenders mit den beschriebenen beiden Teileinheiten kann jedoch auch zweckmäßig sein, um die gleiche ersten Teileinheit mit unterschiedlichen zweiten Teileinheiten zu verwenden. Dies ist insbesondere dann sinnvoll, wenn die fragliche Flüssigkeit von mehreren Personen verwendet wird, die jedoch aus Hygienegründen nicht die gleiche zweite Teileinheit und damit den gleichen Applikator mit Austragöffnung verwenden sollen. Die einfache Entkoppelbarkeit und Koppelbarkeit der Teileinheiten gestattet es, die erste Teileinheit von einer anderen zweiten Teileinheit zu trennen und an die eigene zweite Teileinheit zu koppeln. Wenn eine solche Nutzung des Flüssigkeitsspenders und seiner Teileinrichtungen beabsichtigt ist, wird es als vorteilhaft angesehen, wenn die zweite Teileinheit und insbesondere die hierzu gehörende Austragvorrichtung mit einem wechselbaren Personalisierungselement ausgebildet ist, welches die Identifizierung der eigenen zweiten Teileinheit erleichtert. Insbesondere kann es sich dabei um einen Ring handeln, der auf ein Gehäuse der Austragvorrichtung aufgeschoben ist. Damit hierdurch unterschiedliche zweite Teileinheiten unterscheidbar sind, weisen diese vorzugsweise Personalisierungselemente unterschiedlicher Farben, beispielsweise rote, grüne, blaue oder gelbe Personalisierungselemente, auf. Ähnliches ist beispielsweise aus dem Bereich der elektrischen Zahnbürsten bekannt.

Die Tatsache, dass die Pumpeinrichtung Teil der ersten Teileinheit ist, erleichtert den beschriebenen Wechsel. Während ein Austausch nurdes Flüssigkeitsspeichers, nicht aber der Pumpeinrichtung, mit der Gefahr einherginge, dass die Flüssigkeit während des Wechsels verschüttet wird oder Verunreinigungen eintreten, ist die Handhabung der ersten Teileinheit einschließlich der dortigen Pumpeinrichtung weitgehend gefahrlos. Zudem wäre bei einem Wechsel des Flüssigkeitsspeichers ohne die Pumpeinrichtung eine ausreichend zuverlässige Abdichtung der Pumpeinrichtung am Speicherbehälter nurschwerzu gewährleisten. Dadurch, dass die Pumpeinrichtung fest und vorzugsweise auch in nicht werkzeuglos lösbarer Art und Weise mit dem Speicherbehälter verbunden ist, bleibt die hermetische Abdichtung zwischen dem Speicherbehälter und einem Gehäuse der Pumpeinrichtungunbeeinträchtigt erhalten.

Ein weiterer Vorteil der Pumpeinrichtung als Teil der ersten Teileinheit liegt darin, dass hierdurch die Materialwahl der zweiten Teileinheit einheitlicher sein kann. Während die Pumpeinrichtung aufgrund der Ventile oder anderer Komponenten üblicherweise schwer aus Kunststoffen nur einer Kunststoffgruppe herstellbar ist, kann die zweite Teileinheit ohne Pumpeinrichtung aus solchen einheitlichen Kunststoffen bestehen, insbesondere aus Kunststoffen der Kunststoffgruppen der Polyethylene, der Polypropylene oder der Polyolefine. Dies erleichtert das Recycling der zweiten Teileinheit.

Da sowohl die Verwendung einer zweiten Teileinheit mit wechselnden ersten Teileinheiten als auch die Verwendung einer ersten Teileinheit mit wechselnden zweiten Teileinheiten von Vorteil sein kann, umfasst die Erfindung auch jeweilige Sets, entweder mit mindestens einer ersten Teileinheit und mindestens zwei zweiten Teileinheiten oder mit mindestens einer zweiten Teileinheit und mindestens zwei ersten Teileinheiten.

Die beiden Teileinheiten bilden nach Kopplung durch ihre jeweiligen Außenflächen vorzugsweise gemeinsam eine Außenkontur des Flüssigkeitsspenders. Dies bedeutet insbesondere, dass der Speicherbehälter des Flüssigkeitsspeichers vorzugsweise in von außen sichtbarer Weise an der Austragvorrichtung angekoppelt ist.

Alternativ hierzu kann jedoch auch vorgesehen sein, dass die zweite Teileinheit einen Aufnahmeraum aufweist, der nach Abnahme oder Wegklappen eines gegenüber der Basis beweglichen Gehäuseteils zugänglich ist und der zur Aufnahme der ersten Teileinheit ausgebildet ist. Zum Zwecke des Wechsels der ersten Teileinheit wird dieser Aufnahmeraum geöffnet, beispielsweise durch Abschrauben eines kappenartigen Gehäuseteils, und die erste Teileinheit wird an die Austragvorrichtung der zweiten Teileinheit angekoppelt. Anschließend wird das Gehäuseteil wieder an der zweiten Teileinheit befestigt, bspw. festgeschraubt, und der Flüssigkeitsspender ist verwendbar. Die erste Teileinheit ist von außen nun nicht mehr sichtbar. Sie kann dementsprechend äußerlich unter rein technischen Aspekten gestaltet sein und muss keine oder nur geringe ästhetische Anforderungen erfüllen.

Im Falle der beschriebenen Gestaltung mit einem Aufnahmeraum kann die sichere Kopplung bereits dadurch erzielt werden, dass die erste Teileinheit nur in definierter Ausrichtung in den Aufnahmeraum eingesetzt werden kann und durch das abnehmbare oder bewegliche Gehäuseteil nach Wiederanbringung dessen in Position gehalten wird. Das Gehäuseteil bildet somit einen Teil der Kopplungseinrichtung.

Im Falle einer Gestaltung, bei der die zweite Teileinheit nicht in einem Aufnahmeraum angeordnet ist, ist die Kopplungseinrichtung dagegen vorzugsweise als Bajonettkopplungseinrichtung, als Gewindekopplungseinrichtung, als Klemmkopplungseinrichtung oder als Rastkopplungseinrichtung ausgebildet, wobei die Kopplungseinrichtung ausreichend fest ist, dass bei regelmäßiger Handhabung eine unbeabsichtigte Trennung nicht zu befürchten ist.

Insbesondere bei Flüssigkeitsspendern für Flüssigkeiten, die für Kinder eine Gefahr darstellen könnten, ist es von Vorteil, wenn der Flüssigkeitsspender eine Schutzkappe aufweist, de die Austragöffnung überdeckt, wenn der Spender nicht in Gebrauch ist. Diese Schutzkappe kann insbesondere als kindergesicherte Schutzkappe ausgebildet sein, beispielsweise als Schutzkappe der Squeeze-and-Turn-Bauweise, wie sie im Dokument EP 3055073 B1 beschrieben ist.

Weiterhin ist es von Vorteil, wenn auch der Auslass der ersten Teileinheit stromabwärts der Pumpeinrichtung im Lieferzustand vor Verwendung geschützt ist, da die erste Teileinheit gegebenenfalls länger unbenutzt gelagert wird und sie möglicherweise auch für Kinder zugänglich ist. Vorzugsweise ist der Auslass daher im Lieferzustand mit einem zumindest teilweise abnehmbaren oder zerstörbaren Verschlusselement versehen.

Bei einer besonders einfachen Gestaltung handelt es sich um einen Folienverschluss, also ein den Auslass dicht überdeckenden Folienabschnitt. Dieser wird entweder manuell entfernt oder im Zuge der Kopplung mittels einer scharfen Kante der zweiten Teileinheit durchstochen oder anderweitig zerstört, so dass Flüssigkeit durch den Auslass austreten kann.

Alternativ kann das Verschlusselement jedoch auch in Art einer abnehmbaren Kappe ausgebildet sein. Dies ermöglicht es, diese Kappe wieder aufzusetzen, wenn die betreffende erste Teileinheit temporär von der zweiten Teileinheit getrennt wird.

Die Kappe kann insbesondere als kindergesicherte Kappe ausgebildet sein, wobei wiederum insbesondere eine Kappe der Squeeze-and-Turn-Bauweise bevorzugt wird, wie sie im Dokument EP 3055073 B1 beschrieben ist. Eine solche Kappe weist ein Gewinde oder ein Bajonett auf und verfügt zusätzlich über einen Rastzahn, der im aufgeschraubten Zustand mit einem Gegenzahn verrastet, so dass es einer radialen Kraftbeaufschlagung der Kappe bedarf, um das Gewinde oder das Bajonett anschließend lösen zu können.

Es kann sinnvoll sein, wenn das Verschlusselement zwei Abschnitte aufweist, die im Lieferzustand einstückig miteinander verbunden sind und die beim Öffnen des Auslasses zerstörungsbehaftet voneinander getrennt werden. Einer dieser Abschnitte verbleibt nach dem Öffnen des Auslasses an der ersten Teileinheit. Der andere Abschnitt, der vorzugsweise kappenförmig ist, wird von dem Auslass getrennt. Wird die erste Teileinheit wieder von der zweiten Teileinheit abgekoppelt und der kappenförmige Abschnitt wieder aufgesetzt, so ist dennoch aufgrund der nicht mehr gegebenen Einstückigkeit zu erkennen, dass die erste Teileinheit bereits geöffnet war. Eine solche Kappe stellt somit einen Originalitätsindikator dar.

Damit die Bewegung der Betätigungshandhabe an der zweiten Teileinheit auf die Pumpeinrichtung der ersten Teileinheit übertragen wird, ist an der Pumpeinrichtung ein Auslassstutzen vorgesehen, der von einem Auslasskanal stromabwärts der Pumpeinrichtung durchdrungen ist. Der Außendurchmesser des Auslassstutzens übersteigt vorzugsweise 5 mm nicht. Korrespondierend zum Auslassstutzen oder zu einer anderweitigen Betätigungsfläche, mittels derer die Pumpeinrichtung der ersten Teileinheit durch die zweite Teileinheit kraftbeaufschlagt werden kann, weist die zweite Teileinheit vorzugsweise ein mit der Betätigungshandhabe gekoppeltes und hierdurch gegenüber der Basis bewegliches Übertragungselement auf, welches zur Betätigung der Pumpeinrichtung ausgebildet ist und welches vorzugsweise ebenfalls rohrförmig ausgebildet und von einem Teilabschnitt des Auslasskanals, der zur Austragöffnung führt, durchdrungen ist. Das Übertragungselement und der Auslassstutzen sind bezüglich ihrer Durchmesser vorzugsweise derart aufeinander angepasst, dass das Übertragungselement auf oder in den Auslassstutzen aufschiebbar bzw. einschiebbar ist und umlaufend mit dem Auslassstutzen in einen abdichtenden Kontakt gelangt.

Damit bei getrennten Teileinheiten der Auslassstutzen nicht einfach eindrückbar ist, ist insbesondere im Sinne der Kindersicherheit vorgesehen, dass eine Schutzblende an der Pumpeinrichtung vorgesehen ist. Diese Schutzblende ist vorzugsweise in Art eines Rohrabschnitts ausgebildet, der den Auslassstutzen umgibt und überragt. Durch einen geringen Innendurchmesser von vorzugsweise weniger als 6 mm wird verhindert, dass Kinder allein mit ihren Fingern den Auslassstutzen erreichen und niederdrücken können.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 bis 4 zeigen ein erstes Ausführungsbeispiel eines erfindungsgemäßen Flüssigkeitsspenders vor und während der Montage durch den Endverbraucher sowie bei Verwendung.
Fig. 5 zeigt ein zweites Ausführungsbeispiel eines erfindungsgemäßen Flüssigkeitsspenders.
Fig. 6 und 7 zeigen ein drittes Ausführungsbeispiel eines erfindungsgemäßen Flüssigkeitsspenders.
Fig. 8 und 9 zeigen zwei unterschiedliche Sets, jeweils umfassend einen erfindungsgemäßen Flüssigkeitsspender.

### DETAILLIERTE BESCHREIBUNG DER AUSFÜHRUNGSBEISPIELE

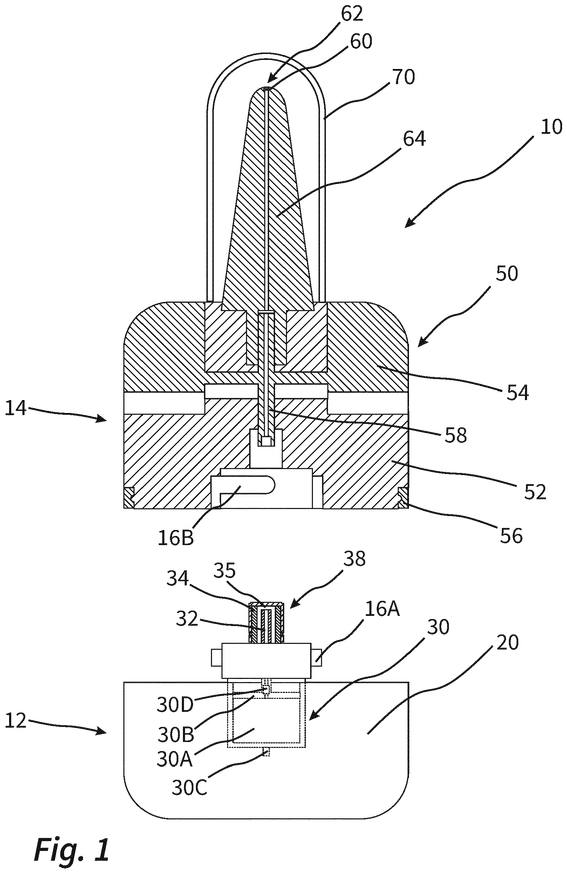

Die drei Ausführungsbeispiele der Fig. 1 bis 7 zeigen verschiedene Besonderheiten, die sie von den jeweils anderenAusführungsbeispielen unterscheiden. Die jeweilige Kombination ist jedoch willkürlich, so dass hiermit alle nachfolgend erörterten Besonderheiten in beliebiger Kombination offenbart sind.

Die Fig. 1 zeigt einen ersten erfindungsgemäßen Flüssigkeitsspender 10 in einem Lieferzustand. Der Flüssigkeitsspender 10 weist zwei Teileinheiten 12, 14 auf, die bestimmungsgemäß vom Endkunden zusammengefügt werden, um die Betriebsbereitschaft des Flüssigkeitsspenders 10 herzustellen.

Die erste Teileinheit 12 verfügt über einen Flüssigkeitsspeicher 20 sowie über eine Pumpeinrichtung 30. Der Flüssigkeitsspeicher kann mit einer Flüssigkeit mit Nikotin oder mit einem Cannabinoid, insbesondere CBD oder THC, als Inhaltsstoff befüllt. Der Flüssigkeitsspeicher 20 kann jedoch auch mit einer pharmazeutischen Flüssigkeit befüllt sein. Diese kann beispielsweise einen oder mehrere der folgenden Wirkstoffe bzw. Inhaltsstoffe enthalten: Acetylcystein, Aciclovir, Aganocide, Albuterol/Salbutamol, Allergenextrakte, Ambroxol, Azelastin, Azelastin, Beclometasondipropionat, Benzethoniumchlorid, Benzocain, Benzydamin, Bepotastin, Betaglucan, Biclotymol, Bromhexin, Budesonid, Formoterol, Carbaethopendeciniumbromid, Cetylpyridinium, Chlorhexidin, Ciclesonid, Cromonen, Dexamethason, Dexamethason, Tramazolin, Dextromethophan, Dimetinden, Docosanol, Ephedrin, Epinephrin, Fenoterol, Flufirvitid, Flunisolid, Fluocinolon, Fluticason, Fluticason-Furoat, Formoterol, Beclometason, Grippeimpfstoff, Guaifenesin, Hexamidin, Hexetidin, Indacaterol, Interferon, lodiopovidon, Ipratropium, Ipratropium, Ipratropiumbromid, Isländisch Moos, Isoprenalin, Ketotifen, lebendige abgeschwächtes Viren, Levocabastin, Locabastin, Loratadin, Milben-Extrakt, Mometason, Mucinex, Naphazolin, Nasal-Gel, Natürliche Heilmittel wie Pflanzenextrakt, TCM, Homöopathie und Bienenextrakt, Nedocromil, Neuraminidase-Hemmer, Norovirus-Impfstoff, Nostrilla, Olopatadin, Oxymethazolin, Phenylephrin, Phenylpropanolamin, Pirbuterol, Pleconaril, Prednisolon, Procaterol, Promethazin, Pseudoephedrin, Salmeterol, Terbutalin, Tetryzolin, Tibezonium, Tiotropium, Tixocortol, Tramazolin-Hydrochlorid, Triamcinolon, Triamcinolonacetonid, Tuaminoheptan, Tyrothricin, Xylometazolin, Zanamivir und/oder Zinkgluconat.

Die Pumpeinrichtung 30 verfügt über eine volumetrisch veränderliche Pumpkammer 30A, die eingangsseitig durch ein bei Unterdruck in der Pumpkammer 30Aöffnendes Einlassventil30C und ausgangsseitig durch ein bei Überdruck in der Pumpkammer 30Aöffnendes Auslassventil30D begrenzt ist. Die Pumpkammer ist weiterhin durch einen Kolben 30B begrenzt, der zum Zwecke der Verkleinerung und Vergrößerung der Pumpkammer beweglich ist.

Am oberen Ende der Teileinheit 12 ist ein Auslass 35 vorgesehen. Dieser Auslass 35 wird durch eine umlaufende zylindrische Schutzblende 34 und einen darin angeordneten Auslassstutzen 32 gebildet. In nicht näher dargestellter Art und Weise ist der Auslassstutzen 32 mit dem Kolben 30B verbunden, so dass durch Niederdrücken des Auslassstutzens 32 Flüssigkeit aus der Pumpkammer30A gefördert werden kann, die dann durch den Auslassstutzen 32 nach oben steigt. Im Lieferzustand der Fig. 1 ist der Auslass 35 durch eine Kappe 38 überdeckt, so dass zunächst kein Zugang zum Auslass 35 besteht.

Die zweite Teileinheit 14 bildet eine Austragvorrichtung, durch die die Flüssigkeit bestimmungsgemäß abgegeben wird und die der Betätigung der Pumpeinrichtung30 dient. Die Teileinheit 14 weist zu diesem Zweck eine Basis 52 auf, an der ein Nasalapplikator 64 angebracht ist. Am distalen Ende des Nasalapplikators 64 ist die Austragöffnung 62 vorgesehen, wobei diese bei der vorliegenden Gestaltungdurch ein Düsenplättchen 60 mit einer Vielzahl sehr kleiner Durchbrechungen gebildet wird. Der Nasalapplikator 64 ist im Zustand der Fig. 1 durch eine lediglich schematisch dargestellte Schutzkappe 70 überdeckt.

Gegenüber der Basis 52 ist eine Betätigungshandhabe 54 beweglich, die fest mit einem rohrförmigen Übertragungselement 58 verbunden ist. Das Übertragungselement 58 wird von einem Teilabschnitt eines Austragkanals durchdrungen. Im Bereich des Nasalapplikators 64 ist eine gleitende Dichtung zwischen dem Übertragungselement 58 und dem Nasalapplikator 64 vorgesehen, so dass Flüssigkeit, die durch das Übertragungselement 58 nach oben steigt, in einen weiteren Teilabschnitt des Auslasskanals innerhalb des Nasalapplikators 64 und somit zur Austragöffnung 62 strömen kann.

Am äußeren Umfang des Gehäuses der zweiten Teileinheit 14 ist ein Personalisierungsring 56 vorgesehen. Es handelt sich um einen farbigen Kunststoffring, der es gestattet, durch Wahl des Rings in einer gewünschten Farbe die zweite Teileinheit 14 zu personalisieren. Hierdurch können beispielsweise mehrere Familienmitglieder jeweils eine solche zweite Teileinheit 14 haben, die voneinander unterscheidbar sind.

Am unteren Ende der zweiten Teileinheit 14 sind einander gegenüberliegend zwei Bajonettnuten 16B vorgesehen, die zum Zusammenwirken mit Bajonettnocken 16A der ersten Teileinheit vorgesehen sind. Der Vorgang der Kopplung der Teileinheiten 12, 14 wird im Weiteren noch erläutert.

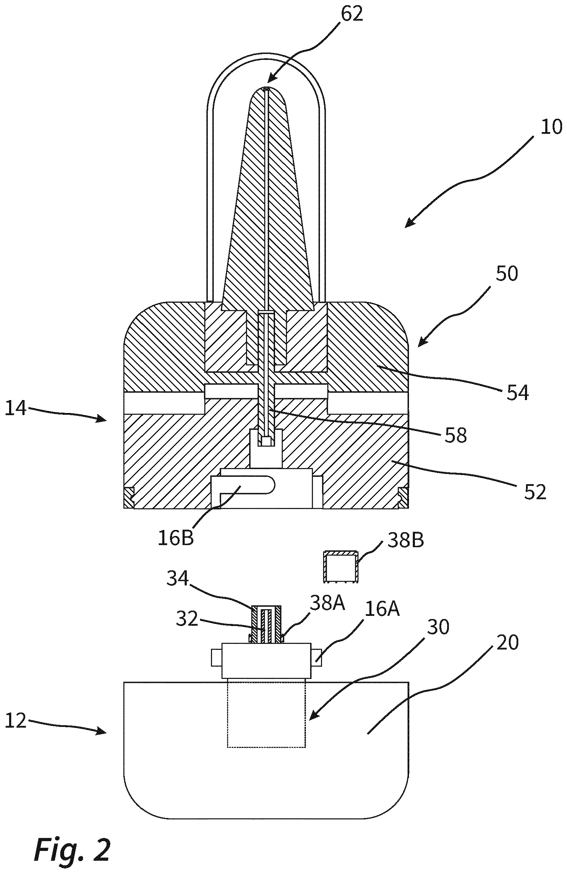

Fig. 2 zeigt den Flüssigkeitsspender 10 der Fig. 1 nach teilweiser Abnahme der Kappe 38. Es ist zu ersehen, dass ein Hauptabschnitt 38B der Kappe 38 entfernt wurde, so dass der Auslassstutzen 32 nun von oben zugänglich ist. Er bleibt aber mit den Fingern, auch mit Kinderfingern, schwer unmittelbar bedienbar, da er unverändert von der Schutzblende 34 geschützt ist.

Ein zweiter Abschnitt 38A der Kappe 38 ist an der Außenseite der Schutzblende 34 verblieben und die zuvor einstückige Verbindung der Abschnitte 38A, 38B ist im Zuge der Abnahme des Hauptabschnitts 38B zerstört worden. Somit kann selbst nach Wiederaufsetzen des Hauptabschnitts 38B auf die Schutzblende 34 unmittelbar ersehen werden, dass die erste Teileinheit 12 zuvor schon geöffnet worden war.

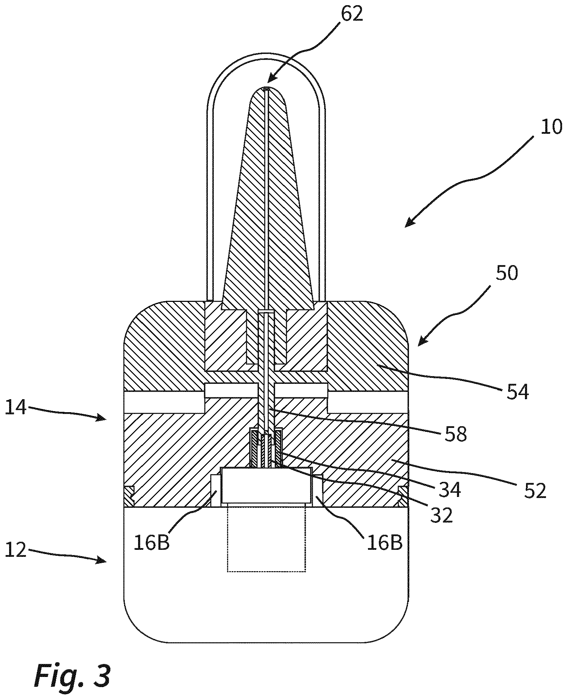

Ausgehend vom Zustand der Fig. 2 erfolgt nun die Koppelung derTeileinheiten 12,14. Hierzu wird in der für ein Bajonett üblichen Art und Weise die erste Teileinheit 12 von unten in die zweite Teileinheit 14 eingeführt, so dass die Bajonettnocken 16A in die Einführnuten der Bajonettnuten 16B einfahren. Anschließend wird die erste Teileinheit 12 um 90° gedreht, um den Koppelungsvorgang abzuschlie-ßen. Fig. 3 zeigt den gekoppelten Zustand. Hieraus ist auch ersichtlich, dass der Auslassstutzen 32 nunmehr in ein offenes Ende des Übertragungselements 58 eingerückt ist, wobei eine umlaufende Abdichtung die Folge ist.

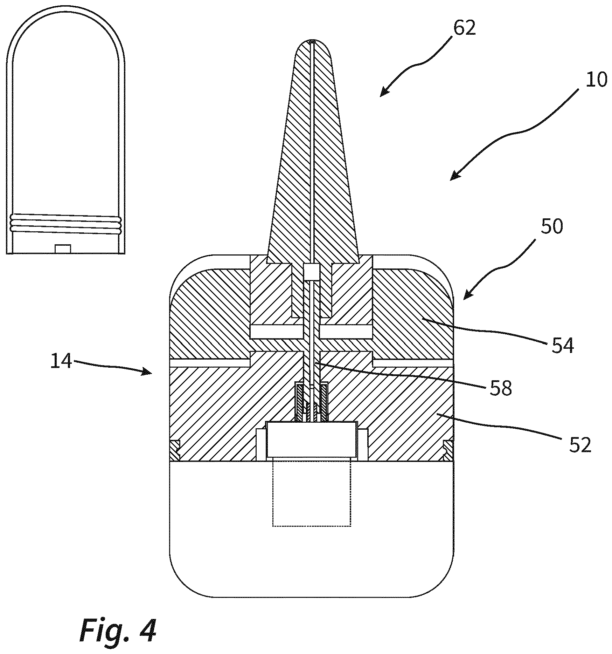

Nun kann nach Abnahme der Kappe 70 der Spender 10 verwendet werden, wie es in Fig. 4 dargestellt ist. Hierfür wird die Betätigungshandhabe 54 gegenüber der Basis 52 niedergedrückt. Die Bewegung wird vom Übertragungselement 58 auf den Auslassstutzen 32 und somit auf den Kolben 30B übertragen, so dass die resultierende Verkleinerung der Pumpkammer 30A den Austrag von Flüssigkeit zur Folge hat.

Sobald der Flüssigkeitsspeicher 20 entleert ist, kann dieser durch Abnehmen der ersten Teileinheit 12 und Ankoppeln einer neuen Teileinheit 12 zusammen mit der Pumpeinrichtung 30 ersetzt werden. Sofern eine andere Person unter hygienisch einwandfreien Bedingungen Flüssigkeit aus dem gleichen Flüssigkeitsspeicher 20 austragen möchte, so kann diese die Teileinheit 12 von der Teileinheit 14 lösen und an ihre eigene zweite Teileinheit 14 ankoppeln.

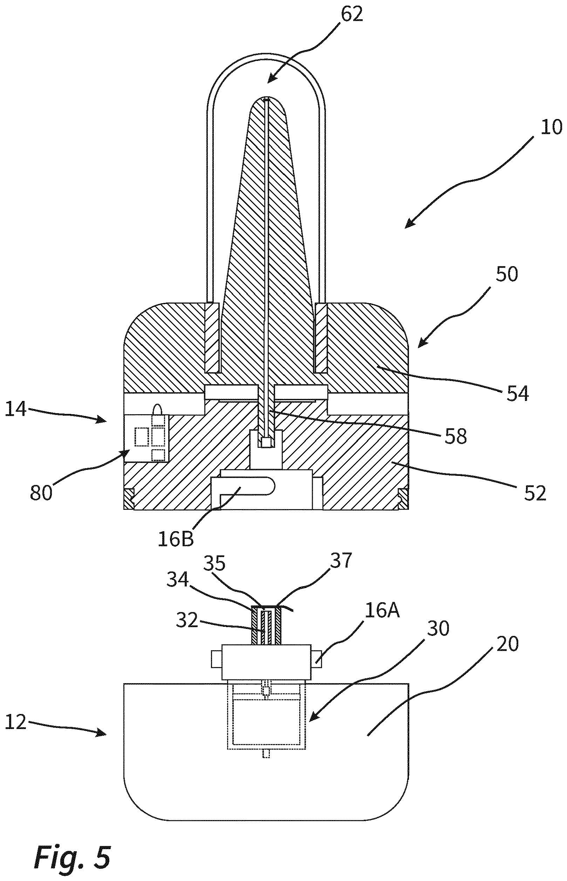

Die Fig. 5 sowie 6 und 7 zeigen alternative Ausgestaltungen, wobei zu beachten ist, dass die dort abweichenden Merkmale auch bei der Gestaltung der Fig. 1 bis 4 vorgesehen sein könnten.

Bei der Gestaltung gemäß Fig. 5 liegt ein erster relevanter Unterschied darin, dass der Nasalapplikator 64 nicht ortsfest zur Basis 52 vorgesehen ist, sondern ortsfest zur Betätigungshandhabe 54 verbleibt. Bei einer solchen Bauform ist insbesondere vorgesehen, dass der Austrag zwar ebenfalls durch Verlagerung der Betätigungshandhabe 54 gegenüber der Basis 52 nach unten erfolgt. Diese Verlagerungwird jedoch insbesondere dadurch bewirkt, dass im gekoppelten Zustand die erste Teileinheit 12 zusammen mit der Basis 52 nach oben gedrückt wird.

Ein zweiter Unterschied liegt darin, dass bei der Gestaltung der Fig. 5 eine elektronische Erfassungseinrichtung 80 vorgesehen ist, die in der Lage ist, die relativbeweglich zwischen der Betätigungshandhabe 54 und der Basis 52 zu erfassen. So können die Austragvorgänge gespeichert und gegebenenfalls über eine drahtlose Schnittstelle an ein externes Gerät wie ein Smartphone weitergegeben werden. Dadurch, dass die zweite Teileinheit 14 wiederverwendbar ist, geht mit dem Vorsehen dieser elektronischen Elemente keine sehr große Umweltbelastung einher.

Ein weiterer Unterschied bei der Gestaltung der Fig. 5 gegenüber der Gestaltung der Fig. 1 bis 4 liegt darin, dass ein Folienverschluss 37 vorgesehen ist, der den Auslass 35 der ersten Teileinheit 12 vor Verwendung verschließt. Dieser Folienverschluss 37 kann manuell entfernt werden, bevor die Kopplung der Teileinheiten 12, 14 stattfindet. Alternativ ist es auch möglich, dass das Übertragungselement 58 an seinem unteren Ende mit ausreichend scharfen Kanten versehen ist, so dass beim Koppeln der Teileinheiten 12, 14 automatisch ein Öffnen des Folienverschlusses 37 stattfindet.

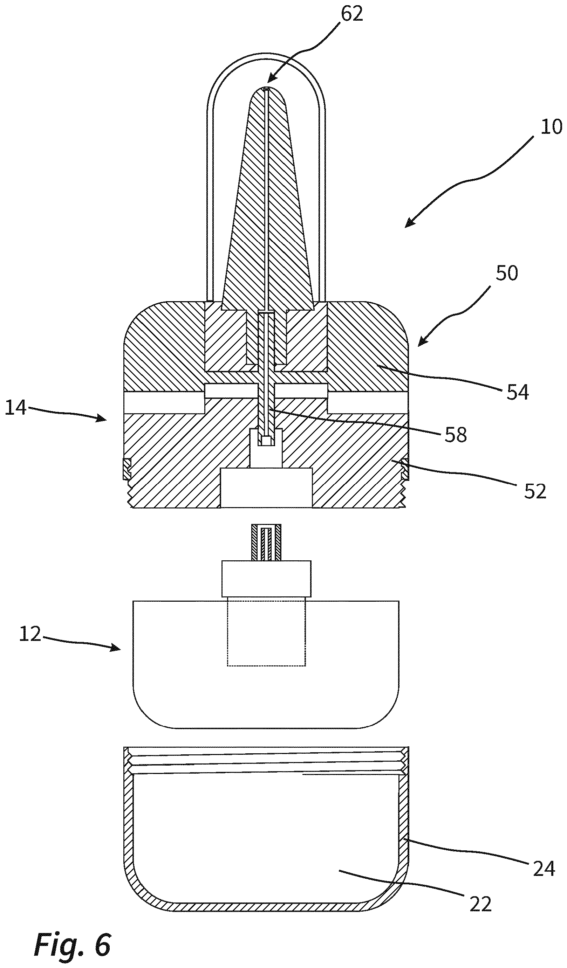

Bei der Gestaltung gemäß der Fig. 6 liegt der wesentliche Unterschied zu den vorangegangenen Gestaltungen darin, dass die zweite Teileinheit 14 ein Gehäuseteil 24 aufweist, welches zusammen mit der Basis 52 einen Aufnahmeraum 22 definiert. Das Gehäuseteil 24 ist mittels einer Gewindeverbindung an der Basis 52 anschraubbar. Der Aufnahmeraum 22 ist dafür ausgebildet, die erste Teileinheit 12 vollständig aufzunehmen.

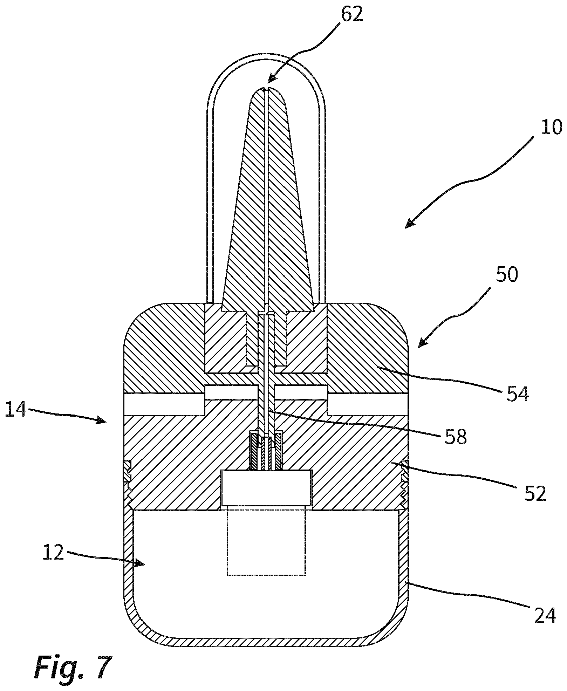

Der gekoppelte Zustand ist in der Fig. 7 dargestellt. Hieraus ist zu ersehen, dass im gekoppelten Zustand die erste Teileinheit 12 vollständig in der zweiten Teileinheit 14 aufgenommen ist. Entsprechend kann auf die Bajonettkopplungseinrichtung der Fig. 1 bis 5 verzichtet werden. Der Boden des Gehäuseteils 24, welches an der Basis 52 angeschraubt ist, sowie der Boden der Teileinheit 12 bilden hier gemeinsam einen Teil der Kopplungseinrichtung, durch die die erste Teileinheit 12 in der für den Austrag passenden Position gehalten wird.

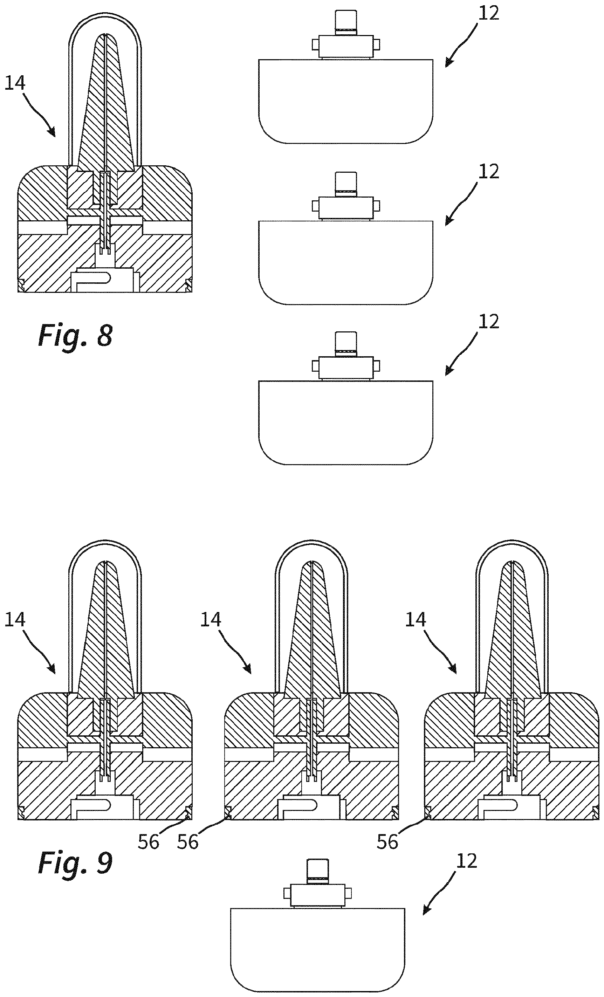

Die Fig. 8 und 9 verdeutlichen zwei Möglichkeiten eines Sets, umfassend einen erfindungsgemäßen Flüssigkeitsspender 10.

Das Set der Fig. 8 weist eine zweite Teileinheit 14 sowie drei erste Teileinheiten 12 auf. Ein solches Set eignet sich insbesondere für einen einzelnen Nutzer, der nacheinander die ersten Teileinheiten 12 an die gleichbleibende zweite Teileinheit 14 ankoppelt. Ist der Flüssigkeitsspeicher 20 einer der Teileinheiten 12 entleert, so wird die jeweils nächste angekoppelt. Auch könnten die Teileinheiten 12 mit verschiedenen Flüssigkeiten befüllt sein, so dass ein Wechsel der ersten Teileinheit 12 auch zweckmäßig sein kann, obwohl die aktuell angekoppelte Teileinheit 12 noch nicht entleert ist.

Fig. 9 zeigt eine andere Gestaltung eines Sets. Hier ist nur eine erste Teileinheit 12 vorgesehen, während das Set 3 zweite Teileinheiten 14 umfasst. Die Teileinheiten 14 weisen vorzugsweise Personalisierungsringe 56 unterschiedlicher Farben auf.

Der bestimmungsgemäße Zweck eines solchen Sets ist es, dass die zweiten Teileinheiten 14 verschiedenen Nutzern, beispielsweise verschiedenen Familienmitgliedern, zugeordnet sind, dass jedoch nur eine einzelne erste Teileinheit 12 benötigt wird, die wahlfrei an eine der zweiten Teileinheiten 14 ankoppelbarist. Wenn ein Nutzer Flüssigkeit austragen will, so koppelt er die gemeinsame erste Teileinheit 12 an seine persönliche Teileinheit 14 und kann anschließend mit dem Austrag beginnen.

## Patentansprüche

1. Flüssigkeitsspender (10) zum Austrag von einer pharmazeutischen Flüssigkeit oder von einer Nikotin und/oder Cannabis enthaltenden Flüssigkeit mit den folgenden Merkmalen:
a. der Flüssigkeitsspender (10) weist einen Flüssigkeitsspeicher (20) auf, und
b. der Flüssigkeitsspender (10) weist eine Austragvorrichtung (50) auf, die über eine mit dem Flüssigkeitsspeicher (20) gekoppelte Basis (52) und eine gegenüber der Basis (52) bewegliche Betätigungshandhabe (54) verfügt, und
c. die Austragvorrichtung (50) weist mindestens eine Austragöffnung (62) auf, durch die Flüssigkeit in eine umgebende Atmosphäre abgegeben werden kann, und
d. der Flüssigkeitsspender (10) weist eine Pumpeinrichtung (30) auf, die über eine volumetrisch veränderliche Pumpkammer (30A) verfügt, wobei die Pumpkammer (30A) über einen Einlasskanal mit Einlassventil (30C) mit dem Flüssigkeitsspeicher (20) verbunden ist und übereinen Auslasskanal mit Auslassventil (30D) mit der Austragöffnung (62) verbunden ist, und
e. die Betätigungshandhabe (54) der Austragvorrichtung (50) ist mit der Pumpeinrichtung (30) gekoppelt, so dass durch Bewegung der Betätigungshandhabe (54) gegenüber der Basis (52) die Pumpeinrichtung (30) betätigt werden kann, und
f. der Flüssigkeitsspender (10) ist als modularer Flüssigkeitsspender ausgebildet, der zwei Teileinheiten (12, 14) aufweist, und
g. eine erste der Teileinheiten (12) weist den Flüssigkeitsspeicher (20) und die Pumpeinrichtung (30) auf, und
h. eine zweite der Teileinheiten (14) weist die Austragvorrichtung (50) und einen Einlass (59) zur Schaffung einer Fluidverbindung mit einem Auslass (35) der ersten Teileinheit (12) auf, und
i. die beiden Teileinheiten (12, 14) verfügen über zusammenwirkende Kopplungsteileinrichtungen (16A, 16B) einer Kopplungseinrichtung (16), um werkzeuglos miteinander gekoppelt und voneinander entkoppelt werden zu können, und
j. die Pumpeinrichtung (30) weist einen vom Auslasskanal durchdrungenen Auslassstutzen (32) auf, mittels dessen das Volumen der Pumpkammer veränderbar ist, **gekennzeichnet durch** das folgende weitere Merkmal:
k. es ist eine Schutzblende (34) an der Pumpeinrichtung (30) vorgesehen, durch die eine unmittelbare manuelle Betätigung der Pumpeinrichtung (30) ohne die Austragvorrichtung (50) unterbunden ist.

2. Flüssigkeitsspender (10) nach Anspruch 1 mit dem folgenden weiteren Merkmal:
a. die zweite Teileinheit (14) weist einen Aufnahmeraum (22) auf, der nach Abnahme oder Wegklappen eines gegenüber der Basis beweglichen Gehäuseteils (24) zugänglich ist und der zur Aufnahme der ersten Teileinheit (12) ausgebildet ist.

3. Flüssigkeitsspender (10) nach Anspruch 1 mit dem folgenden weiteren Merkmal:
a. die beiden Teileinheiten (12, 14) sind derart ausgebildet, dass nach Koppeln derTeileinheiten (12, 14) Außenflächen beider Teileinheiten (12, 14) gemeinsam eine Außenkontur des Flüssigkeitsspenders (10) bilden.

4. Flüssigkeitsspender (10) nach einem der vorstehenden Ansprüche mit dem folgenden weiteren Merkmal:
a. die Pumpeinrichtung (30) ist in nicht werkzeuglos lösbarer Weise mit dem Flüssigkeitsspeicher (20) zur Bildung der ersten Teileinheit (12) verbunden.

5. Flüssigkeitsspender (10) nach einem der vorstehenden Ansprüche mit einem der folgenden weiteren Merkmale:
a. die Kopplungseinrichtung (16) ist als Bajonettkopplungseinrichtung ausgebildet, oder
b. die Kopplungseinrichtung ist als Gewindekopplungseinrichtung ausgebildet, oder
c. die Kopplungseinrichtung ist als Klemmkopplungseinrichtu ng oder als Rastkopplungseinrichtung ausgebildet.

6. Flüssigkeitsspender (10) nach einem der vorstehenden Ansprüche mit einem der folgenden weiteren Merkmale:
a. die mindestens eine Austragöffnung (62) ist ortsfest zur Betätigungshandhabe (54) angeordnet, oder
b. die mindestens eine Austragöffnung (62) ist ortsfest zur Basis (52) angeordnet.

7. Flüssigkeitsspender (10) nach einem der vorstehenden Ansprüche mit dem folgenden weiteren Merkmal:
a. die Austragvorrichtung (50) ist mit einem wechselbaren Personalisierungselement (56) ausgebildet,
vorzugsweise mit einem der folgenden zusätzlichen Merkmale:
b. das Personalisierungselement (56) ist in Art eines Rings (56) ausgebildet, der an einer Außenseite eines Gehäuses der Austragvorrichtung (50) auf das Gehäuse aufgeschoben ist, und/oder
c. das Personalisierungselement (56) weist eine rote, grüne, blaue oder gelbe Farbgebung auf.

8. Flüssigkeitsspender (10) nach einem der vorstehenden Ansprüche mit dem folgenden weiteren Merkmal:
a. der Auslass (35) der ersten Teileinheit weist im Lieferzustand ein zumindest teilweise abnehmbares oder zerstörbares Verschlusselement (37, 38) auf,
insbesondere mit mindestens einem der folgenden zusätzlichen Merkmale:
b. das Verschlusselement (37) ist als Folienverschluss (37) ausgebildet, der den Auslass überdeckt und der vor dem Koppeln an die zweite Teileinheit (14) abnehmbar oder von der zweiten Teileinheit (14) zerstörbar ausgebildet ist, oder
c. das Verschlusselement (38) ist in Art einer abnehmbaren Kappe (38) ausgebildet, insbesondere einer Kappe mit einem Kindersicherungsmechanismus, insbesondere vorzugsweise nach dem Squeeze-and-Turn-Prinzip, oder
d. das Verschlusselement (38) weist zwei Abschnitte (38A, 38B) auf, die im Lieferzustand einstückig miteinander verbunden sind und die beim Öffnen des Auslasses zerstörungsbehaftet voneinander getrennt werden.

9. Flüssigkeitsspender (10) nach einem der vorstehenden Ansprüche mit dem folgenden weiteren Merkmal:
a. die zweite Teileinheit (14) weist ein mit der Betätigungshandhabe (54) gekoppeltes und hierdurch gegenüber der Basis (52) bewegliches Übertragungselement (58) auf, welches zur Betätigung der Pumpeinrichtung (30) der ersten Teileinheit (12) ausgebildet ist,
vorzugsweise mit mindestens einem der folgenden zusätzlichen Merkmale:
b. das Übertragungselement (58) ist von einem Teilabschnitt des Auslasskanals durchdrungen und gelangt im Zuge der Kopplung der Teileinheiten (12, 14) in umlaufend abgedichteten Kontakt mit einem Teilabschnitt des Auslasskanals an der ersten Teileinheit (12), und/oder
c. das Übertragungselement (58) ist abschnittsweise in Art eines Rohrabschnitts ausgebildet, der einen Außendurchmesser von maximal 5 mm aufweist.

10. Flüssigkeitsspender (10) nach einem der vorstehendenAnsprüche mit dem folgenden weiteren Merkmal:
a. die Basis (52) der Austragvorrichtung (50) sowie die Betätigungshandhabe (54) sind aus Materialien dergleichen Kunststoffgruppe hergestellt,
insbesondere vorzugsweise mit einem der folgenden zusätzlichen Merkmale:
b. die Kunststoff-Gruppe, aus der die Basis (52) und die Betätigungshandhabe (54) hergestellt sind, ist die Kunststoff-Gruppe der Polyethylene, oder
c. die Kunststoff-Gruppe, aus der die Basis (52) und die Betätigungshandhabe (54) hergestellt sind, ist die Kunststoff-Gruppe der Polypropylene, oder
d. die Kunststoff-Gruppe, aus der die Basis (52) und die Betätigungshandhabe (54) hergestellt sind, ist die Kunststoff-Gruppe der Polyolefine.

11. Flüssigkeitsspender (10) nach einem der vorstehenden Ansprüche mit mindestens einem der folgenden Merkmale:
a. es sind eine Mehrzahl von Austragöffnungen (62) vorgesehen, die vorzugsweise als Durchbrechungen in einem gemeinsamen Düsenelement (60) ausgebildet sind, und/oder
b. es ist eine von der Austragvorrichtung (50) abnehmbare und wiederaufsetzbare Kappe (70) vorgesehen, insbesondere vorzugsweise eine kindergesicherte Kappe, und/oder
c. der Flüssigkeitsspeicher (20) weist ein Volumen zwischen 5 ml und 50 ml auf, insbesondere ein Volumen zwischen 10 ml und 20 ml, und/oder
d. der Flüssigkeitsspeicher (20) ist mit einer pharmazeutischen Flüssigkeit oder mit einer Nikotin und/oder Cannabis enthaltenden Flüssigkeit befüllt, und/oder
e. die Austragöffnung (62) ist an einem Nasalapplikator vorgesehen, oder
f. die Austragöffnung (62) ist an einem Oralapplikatorvorgesehen, und/oder
g. die Kopplungsteileinrichtung (16B) der zweiten Teileinheit (14) ist an der Basis (52) vorgesehen.

12. Flüssigkeitsspender (10) nach einem der vorstehenden Ansprüche mit dem folgenden Merkmal:
a. die zweite Teileinheit (14) ist mit einerelektronischen Erfassungseinrichtung (80) zur Erfassung der Handhabung des Flüssigkeitsspenders ausgestattet,
vorzugsweise mit dem folgenden zusätzlichen Merkmal:
b. die elektronische Erfassungseinrichtung (80) ist zur Erfassung der Betätigung des Flüssigkeitsspenders (10) und/oder zur Erfassung eines Flüssigkeitsaustrags aus dem Flüssigkeitsspender (10) ausgebildet.

13. Set mit dem folgenden Merkmal:
a. das Set umfasst eine erste Teileinheit (12) und eine zweite Teileinheit (14) eines Flüssigkeitsspenders (10) nach einem der Ansprüche 1 bis 12,
**gekennzeichnet durch** das folgende zusätzliche Merkmal:
b. das Set umfasst weiterhin mindestens eine weitere zweite Teileinheit (14).

14. Set mit dem folgenden Merkmal:
a. das Set umfasst eine erste Teileinheit (12) und eine zweite Teileinheit (14) eines Flüssigkeitsspenders (10) nach einem der Ansprüche 1 bis 12,
**gekennzeichnet durch** das folgende zusätzliche Merkmal:
b. das Set umfasst weiterhin mindestens eine weitere erste Teileinheit (12).

## Claims

1. Liquid dispenser (10) for discharging a pharmaceutical liquid or a liquid containing nicotine and/or cannabis, having the following features:
a. the liquid dispenser (10) comprises a liquid reservoir (20), and
b. the liquid dispenser (10) comprises a discharge apparatus (50), which has a base (52) coupled to the liquid reservoir (20) and an actuator (54) which is movable relative to the base (52), and
c. the discharge apparatus (50) has at least one discharge opening (62), through which liquid can be released into an ambient atmosphere, and
d. the liquid dispenser (10) comprises a pump device (30) which has a volumetrically variable pump chamber (30A), the pump chamber (30A) being connected to the liquid reservoir (20) via an inlet channel with an inlet valve (30C) and being connected to the discharge opening (62) via an outlet channel with an outlet valve (30D), and
e. the actuator (54) of the discharge apparatus (50) is coupled to the pump device (30), such that moving the actuator (54) relative to the base (52) makes it possible to actuate the pump device (30), and
f. the liquid dispenser (10) is in the form of a modular liquid dispenser having two sub-units (12, 14), and
g. a first one of the sub-units (12) comprises the liquid reservoir (20) and the pump device (30), and
h. a second one of the sub-units (14) comprises the discharge apparatus (50) and an inlet (59) for providing a fluid connection with an outlet (35) of the first sub-unit (12), and
i. the two sub-units (12, 14) have interacting coupling sub-devices (16A, 16B) of a coupling device (16), in order to be able to be coupled to and decoupled from one another without tools, and
j. the pump device (30) has an outlet port (32), through which the outlet channel passes and by means of which the volume of the pump chamber can be varied,
**characterized by** the following further feature:
k. a protective covering element (34) is provided on the pump device (30) and prevents direct manual actuation of the pump device (30) without the discharge apparatus (50).

2. Liquid dispenser (10) according to Claim 1, having the following further feature:
a. the second sub-unit (14) has a receiving space (22), which is accessible after a housing part (24), which can be moved relative to the base, has been removed or folded back and is designed to receive the first sub-unit (12).

3. Liquid dispenser (10) according to Claim 1, having the following further feature:
a. the two sub-units (12, 14) are designed such that, after the sub-units (12, 14) have been coupled, outer surfaces of the two sub-units (12, 14) together form an outer contour of the liquid dispenser (10).

4. Liquid dispenser (10) according to one of the preceding claims, having the following further feature:
a. the pump device (30) is connected to the liquid reservoir (20) to form the first sub-unit (12) such that detachment without tools is not possible.

5. Liquid dispenser (10) according to one of the preceding claims, having one of the following further features:
a. the coupling device (16) is in the form of a bayonet coupling device, or
b. the coupling device is in the form of a threaded coupling device, or
c. the couping device is in the form of a clamping coupling device or a latching coupling device.

6. Liquid dispenser (10) according to one of the preceding claims, having one of the following further features:
a. the at least one discharge opening (62) is stationary relative to the actuator (54), or
b. the at least one discharge opening (62) is stationary relative to the base (52).

7. Liquid dispenser (10) according to one of the preceding claims, having the following further feature:
a. the discharge apparatus (50) is designed with an interchangeable customization element (56),
preferably having one of the following additional features:
b. the customization element (56) is designed in the manner of a ring (56), which is pushed on the housing on an outer side of a housing of the discharge apparatus (50), and/or
c. the customization element (56) has a red, green, blue or yellow colouring.

8. Liquid dispenser (10) according to one of the preceding claims, having the following further feature:
a. the outlet (35) of the first sub-unit has, in the state of delivery, an at least partially removable or destructible closure element (37, 38),
in particular having at least one of the following additional features:
b. the closure element (37) is in the form of a film closure (37), which covers the outlet and can be removed before the coupling to the second sub-unit (14) or destroyed by the second sub-unit (14), or
c. the closure element (38) is designed in the manner of a removable cap (38), in particular a cap with a child safety mechanism, in particular preferably according to the squeeze-and-turn principle, or
d. the closure element (38) has two portions (38A, 38B), which are connected to one another in one piece in the state of delivery and are separated from one another, being destroyed in the process, on opening the outlet.

9. Liquid dispenser (10) according to one of the preceding claims, having the following further feature:
a. the second sub-unit (14) has a transfer element (58), which is coupled to the actuator (54) and as a result can be moved relative to the base (52), and is designed to actuate the pump device (30) of the first sub-unit (12),
preferably having at least one of the following additional features:
b. a portion of the outlet channel passes through the transfer element (58), which in the course of coupling the sub-units (12, 14) comes into circumferentially sealed contact with a portion of the outlet channel on the first sub-unit (12), and/or
c. the transfer element (58) is partially formed in the manner of a tube portion, which has an outside diameter of at most 5 mm.

10. Liquid dispenser (10) according to one of the preceding claims, having the following further feature:
a. the base (52) of the discharge apparatus (50) and also the actuator (54) are made from materials belonging to the same group of plastics,
in particular preferably having one of the following additional features:
b. the group of plastics from which the base (52) and the actuator (54) are made is the plastics group of polyethylenes, or
c. the group of plastics from which the base (52) and the actuator (54) are made is the plastics group of polypropylenes, or
d. the group of plastics from which the base (52) and the actuator (54) are made is the plastics group of polyolefins.

11. Liquid dispenser (10) according to one of the preceding claims, having at least one of the following features:
a. a plurality of discharge openings (62) are provided and are preferably in the form of apertures in a common nozzle element (60), and/or
b. a cap (70), in particular preferably a child safety cap, which can be removed from the discharge apparatus (50) and reused is provided, and/or
c. the liquid reservoir (20) has a volume between 5 ml and 50 ml, in particular a volume between 10 ml and 20 ml, and/or
d. the liquid reservoir (20) is filled with a pharmaceutical liquid or with a liquid containing nicotine and/or cannabis, and/or
e. the discharge opening (62) is provided on a nasal applicator, or
f. the discharge opening (62) is provided on an oral applicator, and/or
g. the coupling sub-device (16B) of the second sub-unit (14) is provided on the base (52).

12. Liquid dispenser (10) according to one of the preceding claims, having the following feature:
a. the second sub-unit (14) is equipped with an electronic detection device (80) for detecting the handling of the liquid dispenser,
preferably having the following additional feature:
b. the electronic detection device (80) is designed to detect the actuation of the liquid dispenser (10) and/or to detect a discharge of liquid from the liquid dispenser (10).

13. Set having the following feature:
a. the set comprises a first sub-unit (12) and a second sub-unit (14) of a liquid dispenser (10) according to one of Claims 1 to 12,
**characterized by** the following additional feature:
b. the set furthermore comprises at least one further second sub-unit (14).

14. Set having the following feature:
a. the set comprises a first sub-unit (12) and a second sub-unit (14) of a liquid dispenser (10) according to one of Claims 1 to 12,
**characterized by** the following additional feature:
b. the set furthermore comprises at least one further first sub-unit (12).

## Revendications

1. Distributeur de liquide (10) pour distribuer un liquide pharmaceutique ou un liquide contenant de la nicotine et/ou du cannabis, avec les caractéristiques suivantes :
a. le distributeur de liquide (10) présente un réservoir de liquide (20), et
b. le distributeur de liquide (10) présente un dispositif de distribution (50) qui dispose d'une base (52) couplée au réservoir de liquide (20) et d'une poignée d'actionnement (54) mobile par rapport à la base (52), et
c. le dispositif de distribution (50) présente au moins un orifice de distribution (62) à travers lequel le liquide peut être déchargé dans une atmosphère environnante, et
d. le distributeur de liquide (10) présente un appareil de pompage (30) qui dispose d'une chambre de pompage à volume variable (30A), la chambre de pompage (30A) étant reliée au réservoir de liquide (20) par l'intermédiaire d'un canal d'entrée avec une soupape d'entrée (30C) et étant reliée à l'orifice de distribution (62) par l'intermédiaire d'un canal de sortie avec une soupape de sortie (30D), et
e. la poignée d'actionnement (54) du dispositif de distribution (50) est couplée à l'appareil de pompage (30), de telle sorte que l'appareil de pompage (30) peut être actionné par déplacement de la poignée d'actionnement (54) par rapport à la base (52), et
f. le distributeur de liquide (10) est configuré sous forme de distributeur de liquide modulaire qui présente deux unités partielles (12, 14), et
g. une première des unités partielles (12) présente le réservoir de liquide (20) et l'appareil de pompage (30), et
h. une deuxième des unités partielles (14) présente le dispositif de distribution (50) et une entrée (59) pour établir une liaison fluidique avec une sortie (35) de la première unité partielle (12), et
i. les deux unités partielles (12, 14) disposent d'appareils d'accouplement partiels coopérants (16A, 16B) d'un appareil d'accouplement (16) pour pouvoir être accouplés et désaccouplés l'un de l'autre sans outil, et
j. l'appareil de pompage (30) présente une tubulure de sortie (32) traversée par le canal de sortie, au moyen de laquelle le volume de la chambre de pompage peut être modifié,
**caractérisé par** la caractéristique supplémentaire suivante :
k. un écran de protection (34) est prévu sur l'appareil de pompage (30), par lequel un actionnement manuel direct de l'appareil de pompage (30) sans le dispositif de distribution (50) est empêché.

2. Distributeur de liquide (10) selon la revendication 1, avec la caractéristique supplémentaire suivante :
a. la deuxième unité partielle (14) présente un espace de réception (22) qui est accessible après avoir enlevé ou rabattu une partie de boîtier (24) mobile par rapport à la base et qui est configuré pour recevoir la première unité partielle (12).

3. Distributeur de liquide (10) selon la revendication 1, avec la caractéristique supplémentaire suivante :
a. les deux unités partielles (12, 14) sont configurées de telle sorte qu'après l'accouplement des unités partielles (12, 14), des surfaces extérieures des deux unités partielles (12, 14) forment ensemble un contour extérieur du distributeur de liquide (10).

4. Distributeur de liquide (10) selon l'une quelconque des revendications précédentes, avec la caractéristique supplémentaire suivante :
a. l'appareil de pompage (30) est relié, de manière non détachable sans outil, au réservoir de liquide (20) pour former la première unité partielle (12).

5. Distributeur de liquide (10) selon l'une quelconque des revendications précédentes, avec l'une quelconque des caractéristiques supplémentaires suivantes :
a. l'appareil d'accouplement (16) est configuré sous forme d'appareil d'accouplement à baïonnette, ou
b. l'appareil d'accouplement est configuré sous forme d'appareil d'accouplement fileté, ou
c. l'appareil d'accouplement est configuré sous forme d'appareil d'accouplement par serrage ou sous forme d'appareil d'accouplement par encliquetage.

6. Distributeur de liquide (10) selon l'une quelconque des revendications précédentes, avec l'une quelconque des caractéristiques supplémentaires suivantes :
a. l'au moins un orifice de distribution (62) est agencé de manière fixe par rapport à la poignée d'actionnement (54) ; ou
b. l'au moins un orifice de distribution (62) est agencé de manière fixe par rapport à la base (52).

7. Distributeur de liquide (10) selon l'une quelconque des revendications précédentes, avec la caractéristique supplémentaire suivante :
a. le dispositif de distribution (50) est configuré avec un élément de personnalisation interchangeable (56),
de préférence avec l'une quelconque des caractéristiques supplémentaires suivantes :
b. l'élément de personnalisation (56) est configuré à la manière d'une bague (56) qui est enfilée sur le boîtier sur un côté extérieur d'un boîtier du dispositif de distribution (50), et/ou
c. l'élément de personnalisation (56) présente une coloration rouge, verte, bleue ou jaune.

8. Distributeur de liquide (10) selon l'une quelconque des revendications précédentes, avec la caractéristique supplémentaire suivante :
a. la sortie (35) de la première unité partielle présente, à l'état de livraison, un élément de fermeture (37, 38) au moins partiellement amovible ou destructible,
notamment avec au moins l'une quelconque des caractéristiques supplémentaires suivantes :
b. l'élément de fermeture (37) est configuré sous forme de fermeture en feuille (37) qui recouvre la sortie et qui est configurée sous forme amovible ou destructible par la deuxième unité partielle (14) avant l'accouplement à la deuxième unité partielle (14), ou
c. l'élément de fermeture (38) est configuré à la manière d'un capuchon amovible (38), notamment d'un capuchon avec un mécanisme de sécurité pour enfants, notamment de préférence selon le principe « presser et tourner », ou
d. l'élément de fermeture (38) présente deux sections (38A, 38B) qui sont reliées entre elles d'un seul tenant à l'état de livraison et qui sont séparées l'une de l'autre de manière destructive lors de l'ouverture de la sortie.

9. Distributeur de liquide (10) selon l'une quelconque des revendications précédentes, avec la caractéristique supplémentaire suivante :
a. la deuxième unité partielle (14) présente un élément de transmission (58) accouplé à la poignée d'actionnement (54) et mobile de ce fait par rapport à la base (52), lequel est configuré pour actionner l'appareil de pompage (30) de la première unité partielle (12),
de préférence avec au moins l'une quelconque des caractéristiques supplémentaires suivantes :
b. l'élément de transmission (58) est traversé par une section partielle du canal de sortie et vient en contact étanche circonférentiel avec une section partielle du canal de sortie sur la première unité partielle (12) au cours de l'accouplement des unités partielles (12, 14), et/ou
c. l'élément de transmission (58) est configuré par sections à la manière d'une section de tube qui présente un diamètre extérieur de 5 mm au maximum.

10. Distributeur de liquide (10) selon l'une quelconque des revendications précédentes, avec la caractéristique supplémentaire suivante :
a. la base (52) du dispositif de distribution (50) ainsi que la poignée d'actionnement (54) sont fabriquées dans des matériaux du même groupe de matières plastiques,
notamment de préférence avec l'une quelconque des caractéristiques supplémentaires suivantes :
b. le groupe de matières plastiques à partir duquel la base (52) et la poignée d'actionnement (54) sont fabriquées est le groupe de matières plastiques des polyéthylènes, ou
c. le groupe de matières plastiques à partir duquel la base (52) et la poignée d'actionnement (54) sont fabriquées est le groupe de matières plastiques des polypropylènes, ou
d. le groupe de matières plastiques à partir duquel la base (52) et la poignée d'actionnement (54) sont fabriquées est le groupe de matières plastiques des polyoléfines.

11. Distributeur de liquide (10) selon l'une quelconque des revendications précédentes, avec au moins l'une quelconque des caractéristiques suivantes :
a. il est prévu une pluralité d'orifices de distribution (62), qui sont de préférence configurés sous forme d'ajours dans un élément de buse commun (60), et/ou
b. il est prévu un capuchon (70) pouvant être retiré et remis en place par le dispositif de distribution (50), notamment de préférence un capuchon de sécurité pour enfants, et/ou
c. le réservoir de liquide (20) présente un volume compris entre 5 ml et 50 ml, notamment un volume compris entre 10 ml et 20 ml, et/ou
d. le réservoir de liquide (20) est rempli d'un liquide pharmaceutique ou d'un liquide contenant de la nicotine et/ou du cannabis, et/ou
e. l'orifice de distribution (62) est prévu sur un applicateur nasal, ou
f. l'orifice de distribution (62) est prévu sur un applicateur oral, et/ou
g. l'appareil de partie d'accouplement (16B) de la deuxième unité partielle (14) est prévu sur la base (52).

12. Distributeur de liquide (10) selon l'une quelconque des revendications précédentes, avec la caractéristique suivante :
a. la deuxième unité partielle (14) est équipée d'un appareil de détection électronique (80) pour détecter la manipulation du distributeur de liquide,
de préférence avec la caractéristique supplémentaire suivante :
b. l'appareil de détection électronique (80) est configuré pour détecter l'actionnement du distributeur de liquide (10) et/ou pour détecter une distribution de liquide hors du distributeur de liquide (10).

13. Ensemble avec la caractéristique suivante :
a. l'ensemble comprend une première unité partielle (12) et une deuxième unité partielle (14) d'un distributeur de liquide (10) selon l'une quelconque des revendications 1 à 12,
**caractérisé par** la caractéristique supplémentaire suivante :
b. l'ensemble comprend en outre au moins une deuxième unité partielle supplémentaire (14).

14. Ensemble avec la caractéristique suivante :
a. l'ensemble comprend une première unité partielle (12) et une deuxième unité partielle (14) d'un distributeur de liquide (10) selon l'une quelconque des revendications 1 à 12,
**caractérisé par** la caractéristique supplémentaire suivante :
b. l'ensemble comprend en outre au moins une première unité partielle supplémentaire (12).
